# EUROPEAN PATENT SPECIFICATION

(11) **EP 2 247 297 B1**
(45) Date of publication and mention of the grant of the patent: **09.01.2019**
(21) Application number: 09707788.7
(22) Date of filing: 02.02.2009
(51) Int. Cl.: A61K 31/198, A61K 38/00, A61P 11/00, A61P 9/12

(54) **THERAPEUTIC TREATMENT FOR LUNG CONDITIONS**
THERAPEUTISCHE BEHANDLUNG VON LUNGENERKRANKUNGEN
TRAITEMENT THÉRAPEUTIQUE DE CONDITIONS PULMONAIRES

(30) Priority: 31.01.2008 US 25157
(43) Date of publication of application: 10.11.2010
(73) Proprietor: Vanderbilt University, Nashville, TN 37212 (US)
(72) Inventor: SUMMAR, Marshall, L., Brentwood TN 37027 (US); BARR, Frederick, E., Nashville TN 37212 (US); FIKE, Candice, D., Nashville TN 37232-0656 (US); ASCHNER, Judy, L., Brentwood TN 37027 (US)
(74) Representative: Isarpatent
(86) International application number: PCT/US2009/032824
(87) International publication number: WO 2009/099998

(56) References cited:
- US-A1- 2004 235 953
- US-A1- 2007 026 448
- NELIN LEIF D ET AL: "L-arginine, but not D-arginine, increases nitric oxide production and vasodilates hypoxic neonatal pig lungs", FASEB JOURNAL, vol. 18, no. 4-5, 2004, page A327, XP009147240, & FASEB MEETING ON EXPERIMENTAL BIOLOGY: TRANSLATING THE GENOME; WASHINGTON, DISTRICT OF COLUMBIA, USA; APRIL 17-21, 2004 ISSN: 0892-6638
- FIKE CANDICE D ET AL: "L-arginine increases nitric oxide production in isolated lungs of chronically hypoxic newborn pigs", JOURNAL OF APPLIED PHYSIOLOGY, vol. 88, no. 5, May 2000 (2000-05), pages 1797-1803, XP002633028, ISSN: 8750-7587
- MITANI YOSHIHIDE ET AL: "PROLONGED ADMINISTRATION OF L-ARGININE AMELIORATES CHRONIC PULMONARY HYPERTENSION AND PULMONARY VASCULAR REMODELING IN RATS", CIRCULATION, LIPPINCOTT WILLIAMS & WILKINS, US, vol. 96, no. 2, 15 July 1997 (1997-07-15), pages 689-697, XP009083116, ISSN: 0009-7322
- FAGAN JULIE M ET AL: "L-arginine reduces right heart hypertrophy in hypoxia-induced pulmonary hypertension", BIOCHEMICAL AND BIOPHYSICAL RESEARCH COMMUNICATIONS, vol. 254, no. 1, 8 January 1999 (1999-01-08), pages 100-103, XP002633029, ISSN: 0006-291X
- MCCAFFREY MARTIN J ET AL: "Effect of L-arginine infusion on infants with persistent pulmonary hypertension of the newborn", BIOLOGY OF THE NEONATE, vol. 67, no. 4, 1995, pages 240-243, XP009147215, ISSN: 0006-3126
- ASCHNER JUDY L: "New therapies for pulmonary hypertension in neonates and children.", PEDIATRIC PULMONOLOGY. SUPPLEMENT 2004 LNKD- PUBMED:15029628, vol. 26, 2004, pages 132-135, XP002633030, ISSN: 1054-187X
- SMITH ET AL: "Nitric oxide precursors and congenital heart surgery: A randomized controlled trial of oral citrulline", JOURNAL OF THORACIC AND CARDIOVASCULAR SURGERY, MOSBY-YEAR BOOK, INC., ST. LOUIS, MO, US, vol. 132, no. 1, 1 July 2006 (2006-07-01), pages 58-65, XP005693517, ISSN: 0022-5223, DOI: DOI:10.1016/J.JTCVS.2006.02.012
- ROMERO M J ET AL: "Therapeutic use of citrulline in cardiovascular disease", CARDIOVASCULAR DRUG REVIEWS 200609 GB LNKD- DOI:10.1111/J.1527-3466.2006.00275.X, vol. 24, no. 3-4, September 2006 (2006-09) , pages 275-290, XP002633031, ISSN: 0897-5957
- "Persistierende fetale Zirkulation (PFC-Syndrom)" In: Ania Carolina Muntau: "Intensivkurs Pädiatrie", 2009, Elsevier Urban & Fischer, München, XP002633032, ISBN: 978-3-437-43392-4 pages 17-18, * page 18, paragraph 1 *
- KINSELLA J P ET AL: "Bronchopulmonary dysplasia", LANCET THE, LANCET LIMITED. LONDON, GB, vol. 367, no. 9520, 29 April 2006 (2006-04-29), pages 1421-1431, XP025094032, ISSN: 0140-6736, DOI: DOI:10.1016/S0140-6736(06)68615-7 [retrieved on 2006-04-29]
- ANANTHAKRISHNAN MADHUMITA ET AL: "L-Citrulline ameliorates chronic hypoxia-induced pulmonary hypertension in newborn piglets", AMERICAN JOURNAL OF PHYSIOLOGY - LUNG CELLULAR AND MOLECULAR PHYSIOLOGY, vol. 297, no. 3, September 2009 (2009-09), pages L506-L511, XP002633033, ISSN: 1040-0605
- SCHREIBER ET AL.: 'Inhaled nitric oxide in premature infants with the respiratory distress syndrome.' N ENGL. J. MED. vol. 349, 2003, pages 2099 - 2107
- BERKENBOSCH ET AL.: 'Decreased synthesis and vasodilation to nitric oxide in piglets with hypoxia induced pulmonary hypertension.' AM. J. PHYSIOL. JUNG CELL. MOL. PHYSIOL. vol. 278, 2000, pages L276 - L283
- ESCOBEDO M B ET AL: "A baboon model of bronchopulmonary dysplasia - I. Clinical features", EXPERIMENTAL AND MOLECULAR PATHOLOGY, ACADEMIC PRESS, US, vol. 37, no. 3, 1 December 1982 (1982-12-01), pages 323-334, XP022973006, ISSN: 0014-4800, DOI: 10.1016/0014-4800(82)90045-4 [retrieved on 1982-12-01]
- ROBERTA A. BALLARD ET AL: "Inhaled Nitric Oxide in Preterm Infants Undergoing Mechanical Ventilation", NEW ENGLAND JOURNAL OF MEDICINE, vol. 355, no. 4, 27 July 2006 (2006-07-27) , pages 343-353, XP055212287, ISSN: 0028-4793, DOI: 10.1056/NEJMoa061088
- ARUL VADIVEL ET AL: "L-Citrulline Attenuates Arrested Alveolar Growth and Pulmonary Hypertension in Oxygen-Induced Lung Injury in Newborn Rats", PEDIATRIC RESEARCH, vol. 68, no. 6, 1 December 2010 (2010-12-01), pages 519-525, XP55473629, US ISSN: 0031-3998, DOI: 10.1203/PDR.0b013e3181f90278

## Description

### TECHNICAL FIELD

The presently disclosed subject matter relates to the treatment of bronchopulmonary dysplasia (BPD), such as in infants.

### BACKGROUND

Bronchopulmonary dysplasia (BPD) typically occurs in infants, particularly preterm infants, and is characterized as an acute injury to the lungs by either oxygen and/or mechanical ventilation, resulting in interference with or inhibition of lung alveolar and vascular development (Jobe et al. (2001) Am J Respir Crit Care Med 163:1723-1729). In animal models, inhaled NO improves both gas exchange and lung structural development, but the use of this therapy in infants at risk for BPD is controversial (Ballard et al. (2006) N Engl J Med 355 :343-353).

Infants with chronic lung disease and cyanotic congenital heart disease frequently suffer from hypoxia. Because of its effects on both existing and developing pulmonary arteries, chronic hypoxia causes progressive changes in both the function and structure of the pulmonary circulation. Shimoda L, et al., Physiol Res (2000) 49:549-560; Subhedar, N. V., Acta Paediatr suppl (2004) 444:29-32. Ultimately, chronic hypoxia results in severe pulmonary hypertension culminating in right-sided heart failure and death.

Nelin, et al. (2004) FASEB Journal 18(4-5): A327, discloses that NO production is impaired in the lungs of piglets exposed to chronic hypoxia. Nelin, page 524. Nelin reports that vasoconstriction due to acute hypoxia in isolated pig lungs is attenuated by perfusing the isolated lung with arginine at 1 mM concentration. Nelin's focus is on comparing the effects of L-arginine and D-arginine.

Fike, et al. (2000) Journal of Applied Physiology 88(5): 1797-1803 reports that perfusion with L-arginine at 10 mM concentration improves pulmonary hypertension in lungs of piglets exposed to chronic hypoxia. Fike, page 1800, Table 3. The goal of administering L-arginine for treating pulmonary hypertension, as discussed in Fike, is to increase NO levels. Fike, page 1802.

Mitani, et al. (1997) Circulation 96(2): 689-697 reports that L-arginine ameliorated the changes associated with pulmonary hypertension in rat models of hypertension, suggested to be due to modification of endogenous NO production. Mitani, p. 694. Mitani reports studies on two models of pulmonary damage using adult rats treated with (1) chronic hypoxia or (2) monocrotaline. Mitani observed the effects of the "Arginine Paradox" which reduces the effectiveness of arginine.

Fagan, et al. (1999) Biochemical and Biophysical Research Communications 254(1): 100-103 discloses that chronic hypoxia results in a decreased release of NO and may contribute to the onset of pulmonary hypertension. Fagan notes that plasma levels of arginine, the substrate for nitric oxide synthase (NOS) were not significantly different during hypoxia, indicating that plasma arginine levels are maintained at normal concentrations during hypoxic exposure. Fagan, page 102.

McCaffrey, et al. (1995) Biology of the Neonate 67(4): 240-243 contemplates the treatment of pulmonary hypertension in newborns by administering intravenous L-arginine in a bolus of 2.87 mmoles/kg. McCaffery cautions that, "[t]he purpose of this pilot study was to evaluate the safety and efficacy of the intravenous administration of *L*-arginine to infants with PPHN." *Id.,* 240-241. McCaffery notes that, "[e]fficacy...must be confirmed in placebo-controlled trials which should also evaluate the safety and benefits of a multiple dose regimen of *L*-arginine." *Id*., 243.

The disclosure of Smith, et al. (2006) Journal of Thoracic and Cardiovascular Surgery 132(1): 58-65 is drawn to reduction of back pressure on the heart to allow the heart to undergo repair. Smith suggests that the administration of citrulline is effective to "prevent post-operative pulmonary hypertension" accordingly this speculation is limited to *post-operative* treatment of a subset of congenital heart surgery patients. Smith, page 64.

Romero, et al. (2006) Cardiovascular Drug Reviews 24(3-4): 275-290 suggests that citrulline has promise as a therapeutic adjunct in disease states associated with arginine deficiencies. Romero, page 277. Like Smith, Romero suggests that citrulline supplementation may have beneficial effects for the treatment of diseases in which arginine supplementation is effective. These diseases include, *inter alia,* systemic hypertension and cardiovascular dysfunction. Romero, pages 284-285.

Ballard, et al. (2006) New England Journal of Medicine, vol. 355, no. 4, p. 343-353 describes that inhaled NO leads to an improvement in BPD.

Accordingly, approaches for the treatment of lung conditions, such as BPD and chronic hypoxia-induced pulmonary hypertension, and further such as in infants, representative a long-felt and continuing need in the art.

### SUMMARY

The presently disclosed subject matter provides a pharmaceutical composition comprising an amount of citrulline effective for use in treating subjects at risk for suffering or suffering from bronchopulmonary dysplasia (BPD).

In some embodiments, the composition is formulated for oral administration, intravenous administration, or a combination thereof.

An object of the presently disclosed subject matter having been stated hereinabove, other objects will become evident as the description proceeds, when taken in connection with the accompanying drawings and examples as best described hereinbelow.

### BRIEF DESCRIPTION OF THE DRAWINGS

Figure 1 is a schematic of the urea cycle.
Figure 2 is a flow diagram of study procedures followed in the Examples.
Figure 3 is a bar graph showing mean pulmonary arterial pressure measurements in control (n=6), chronically hypoxic (n=11), and L-citrulline treated chronically hypoxic (n-6) piglets. All values are mean±SEM. *different from control; ⁺different from chronically hypoxic; p<0.05, ANOVA with post-hoc comparison test.
Figure 4 is a bar graph showing calculated pulmonary vascular resistance in control (n=6), chronically hypoxic (n=11), and L-citrulline treated chronically hypoxic (n=6) piglets. All values are mean±SEM. *different from control; ⁺different from chronically hypoxic; p<0.05, ANOVA with post-hoc comparison test.
Figure 5 is a bar graph showing exhaled Nitric Oxide in control (n=6), chronically hypoxic (n=11), and L-citrulline treated chronically hypoxic (n=5) piglets. All values are mean±SEM. *different from control; ⁺different from chronically hypoxic; p<0.05, ANOVA with post-hoc comparison test.
Figure 6 is a bar graph showing nitrite/nitrate accumulation in lung perfusate in control (n=17), chronically hypoxic (n=9), and L-citrulline treated chronically hypoxic (n=5) piglets. All values are mean±SEM. *different from control; ⁺different from chronically hypoxic; p<0.05, ANOVA with post-hoc comparison test.
Figure 7A is an image of an immunoblot for eNOS protein reprobed for actin for lung tissue from controls (n=3), chronic hypoxic (n=3), and L-citrulline treated chronic hypoxic (n=3) piglets.
Figure 7B is a bar graph showing densitometry of eNOS normalized to actin for lung tissue from controls (n=3), chronic hypoxic (n=3), and L-citrulline treated chronic hypoxic (n=3) piglets.

### DETAILED DESCRIPTION

Preterm births continue to be the major challenge in obstetrics and neonatology, accounting for most of the perinatal mortality and long-term neurologic morbidity among newborns. BPD is one of many complications that can be associated with preterm birth. BPD can be associated with prolonged hospitalization of a preterm infant, multiple rehospitalizations during the first few years of life, and developmental delays. Fortunately, BPD is now infrequent in infants of more than 1,200 g birth weight or with gestations exceeding 30 weeks (Jobe et al. (2001) Am J Respir Crit Care Med 163:1723-1729). The incidence of BPD defined as an oxygen need at 36 weeks postmenstrual age is about 30% for infants with birth weights < 1,000 g (Jobe et al. (2001) Am J Respir Crit Care Med 163:1723-1729). Some of these infants have severe lung disease, requiring ventilation and/or supplemental oxygen for months or even years.

Multiple factors contribute to BPD, and probably act additively or synergistically to promote injury. The traditional view has been that BPD is caused primarily by oxidant- and ventilation-mediated injury (Jobe et al. (2001) Am J Respir Crit Care Med 163:1723-1729). Mechanical ventilation and oxygen can interfere with alveolar and vascular development in preterm infants and has been attributed to the development of BPD (Jobe et al. (2001) Am J Respir Crit Care Med 163:1723-1729). Reduced numbers of alveoli can result in a large decrease in surface area, which has been associated with a decrease in dysmorphic pulmonary microvasculature. These anatomic changes are associated with persistent increases in white blood cells and cytokine levels in airway samples (Jobe et al. (2001) Am J Respir Crit Care Med 163:1723-1729).

Inflammation can also play a role in the development of BPD. Multiple proinflammatory and chemotactic factors are present in the air spaces of ventilated preterm infants, and these factors are found in higher concentrations in the air spaces of infants who subsequently develop BPD (Jobe et al. (2001) Am J Respir Crit Care Med 163:1723-1729). Other factors considered important to the development of BPD include: bombesin-like peptides, hyperoxia, hypoxia, poor nutrition, glucocorticoid treatment and the overexpression of the cytokines tumor necrosis factor-α, TGF-α, IL-6, or IL-11 (Jobe et al. (2001) Am J Respir Crit Care Med 163:1723-1729).

Diagnosing BPD generally comprises monitoring an infant's breathing over the initial weeks of life for signs of delayed lung development and a continued and/or increased dependence upon assisted breathing. Diagnostic tests that can be performed to assist in the diagnosis of BPD can include: blood oxygen tests, chest x-rays, and echocardiograms. BPD has traditionally been diagnosed when an infant requires supplemental oxygen at 36 weeks postmenstrual age. Newer definitions used in diagnosing and defining BPD include specific criteria for 'mild,' 'moderate' and 'severe' BPD (Ryan, R. M. (2006) J Perinatology 26:207-209).

Treating BPD can include a mutli-faceted approach to treating the symptoms of the condition and providing an infant's lungs an opportunity to develop. Currently available treatments can comprise: surfactant administration to improve lung aeration, mechanical ventilators to compensate for respiratory failure, supplemental oxygen to insure adequate blood oxygen, bronchodilator medications to improve airflow in the lungs, corticosteroids to reduce swelling and inflammation of airways, fluid control to avoid pulmonary edema, treatments for *patent ductus arteriosus,* and proper nutrition.

Nitric oxide administration via inhalation has been demonstrated to improve lung development in infant animal models (Ballard et al. (2006) N Engl J Med 355:343-353). However, NO administration via inhalation is controversial for human subjects. Thus, in accordance with some embodiments of the presently disclosed subject matter, administering citrulline to a subject suffering from BPD to thereby increase *in vivo* NO synthesis can provide an alternative to NO inhalation as a BPD treatment.

Because of its effects on both existing and developing pulmonary arteries, chronic hypoxia causes progressive changes in both the function and structure of the pulmonary circulation. Shimoda L, et al., Physiol Res (2000) ;49:549-560; Subhedar, N. V., Acta Paediatr suppl (2004);444:29-32. Ultimately, chronic hypoxia results in severe pulmonary hypertension culminating in right-sided heart failure and death. Currently the therapy for pulmonary hypertension in infants suffering from chronic cardiopulmonary disorders associated with persistent or episodic hypoxia is largely limited to improving the underlying cardiopulmonary disorder and attempts to achieve adequate oxygenation. Abman, S. H.; Arch Dis Child Fetal Neonatal Ed (2002) 87: F15-F18; Allen, J. and ATS subcommittee AoP, Am J Respir Crit Care Med (2003) 168: 356-396; Mupanemunda, R. H., Early Human Development (1997) 47: 247-262; Subhedar, N. V., Acta Paediatr suppl (2004) 444:29-32.

Citrulline is a key intermediate in the urea cycle and in the production of nitric oxide (NO). In the urea cycle, citrulline is a precursor for the *de novo* synthesis of arginine. Arginine can be deaminated via arginase to produce urea, which can subsequently be excreted to rid the body of waste nitrogen, particularly ammonia. Alternatively, arginine can provide for the production of NO via nitric oxide synthase. As such, intact urea cycle function is important not only for excretion of ammonia but in maintaining adequate tissue levels of arginine, the precursor of NO.

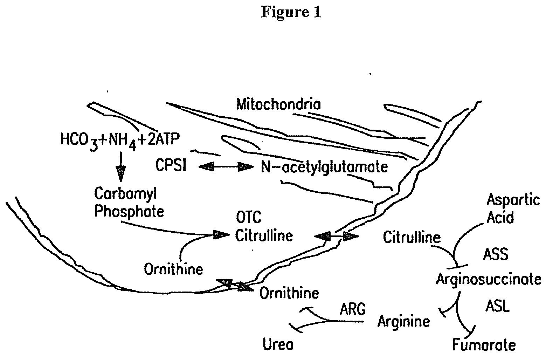

Nitric oxide is synthesized by nitric oxide synthase using arginine as a substrate. The rate-limiting factor in the synthesis of NO is the availability of cellular arginine, and the preferred source of arginine for NO synthesis is de *novo* biosynthesized from citrulline. The *in vivo* synthetic pathway for arginine commences with ornithine. Ornithine is combined with carbamyl phosphate to produce citrulline, which in turn is combined with aspartate, in the presence of adenosine triphosphate, to produce argininosuccinate. In the final step, fumarate is split from argininosuccinate, to produce arginine. The degradative pathway for arginine is by the hydrolytic action of arginase, to produce ornithine and urea. These reactions form the urea cycle. *See also* Figure 1.

As an alternative to degradation for urea synthesis, arginine can provide the substrate necessary for NO synthesis via nitric oxide synthase. Additionally, exogenous citrulline can enter the urea cycle and provide for the *in vivo* synthesis of arginine, which can subsequently provide for NO synthesis. Accordingly, administering citrulline to subjects susceptible to or diagnosed with BPD can increase arginine synthesis and subsequently increase NO production to thereby prevent and/or treat BPD.

### I. THERAPEUTIC METHODS

The presently disclosed subject matter provides a pharmaceutical composition comprising an amount of citrulline effective for use in treating subjects at risk for suffering or suffering from bronchopulmonary dysplasia. The composition may be formulated for oral administration, intravenous administration, or a combination thereof.

The presently disclosed subject matter provides compositions and their use for treating BPD in a subject. In some embodiments, the subject to be treated is a subject suffering from an acute condition associated with BPD. Representative examples of such conditions are disclosed herein above.

Also methods and compositions for treating chronic hypoxia-induced pulmonary hypertension and/or associated complications in a subject are described herein. An effective amount of citrulline or other NO precursor is administered to a subject suffering from chronic hypoxia-induced pulmonary hypertension and/or associated complications and/or at risk for suffering complications associated with chronic hypoxia-induced pulmonary hypertension. The NO precursor may be selected from the group including, but not limited to, citrulline, a precursor that generates citrulline *in vivo,* arginine, a precursor that generates arginine *in vivo,* or combinations thereof. The citrulline or other NO precursor may be administered orally. The citrulline or other NO precursor may be administered intravenously. The subject to be treated may be a subject suffering from an acute condition associated with chronic hypoxia-induced pulmonary hypertension. Representative examples of such conditions are disclosed herein above.

The above nitric oxide precursor may comprise at least one of citrulline, a precursor that generates citrulline *in vivo,* a pharmaceutically acceptable salt thereof, and combinations thereof. See Figure 1. The nitric oxide precursor may be selected from the group including, but not limited to, citrulline, arginine, or combinations thereof. The nitric oxide precursor, such as citrulline, may be administered orally. The nitric oxide precursor, such as citrulline, may be administered intravenously.

In some embodiments, the subject suffers from hypocitrullinemia. In some embodiments the hypocitrullinemia is characterized by plasma citrulline levels of ≤ 37 µmol/liter, in some embodiments, ≤ 25 µmol/liter, in some embodiments, ≤ 20 µmol/liter, in some embodiments, ≤ 10 µmol/liter, in some embodiments, ≤ 5 µmol/liter.

In some embodiments, the subject suffering from a condition as disclosed herein suffers from relative hypocitrullinemia. The term "relative hypocitrullinemia" refers to a state in which the subject suffering from a condition has reduced plasma citrulline as compared to a subject not suffering from a condition.

As used herein, the phrase "treating" refers to both intervention designed to ameliorate a condition in a subject (*e.g*., after initiation of a disease process or after an injury), to ameliorate complications related to the condition in the subject, as well as to interventions that are designed to prevent the condition from occurring in the subject. Stated another way, the terms "treating" and grammatical variants thereof are intended to be interpreted broadly to encompass meanings that refer to reducing the severity of and/or to curing a condition, as well as meanings that refer to prophylaxis. In this latter respect, "treating" can refer to "preventing" to any degree, such as but not limited to in a subject at risk for suffering a condition, or otherwise enhancing the ability of the subject to resist the process of the condition.

The subject treated in the presently disclosed subject matter in its many embodiments is desirably a human subject, although it is to be understood that the principles of the presently disclosed subject matter indicate that the presently disclosed subject matter is effective with respect to all vertebrate species, including warm-blooded vertebrates such as mammals and birds, which are intended to be included in the term "subject". In this context, a mammal is understood to include any mammalian species in which treatment is desirable, such as but not limited to agricultural and domestic mammalian species.

Thus, provided is the treatment of mammals such as humans, as well as those mammals of importance due to being endangered (such as Siberian tigers), of economical importance (animals raised on farms for consumption by humans) and/or social importance (animals kept as pets or in zoos) to humans, for instance, carnivores other than humans (such as cats and dogs), swine (pigs, hogs, and wild boars), ruminants (such as cattle, oxen, sheep, giraffes, deer, goats, bison, and camels), and horses. Also provided is the treatment of birds, including the treatment of those kinds of birds that are endangered, kept in zoos, as well as fowl, and more particularly domesticated fowl, i.e., poultry, such as turkeys, chickens, ducks, geese, guinea fowl, and the like, as they are also of economical importance to humans. Thus, provided is the treatment of livestock, including, but not limited to, domesticated swine (pigs and hogs), ruminants, horses, poultry, and the like.

### II. PHARMACEUTICAL COMPOSITIONS

An effective dose of a composition of the presently disclosed subject matter is administered to a subject in need thereof. An "effective amount" is an amount of a composition sufficient to produce a measurable response (*e.g*., a biologically or clinically relevant response in a subject being treated). Actual dosage levels of active ingredients in the compositions of the presently disclosed subject matter can be varied so as to administer an amount of the active compound(s) that is effective to achieve the desired therapeutic response for a particular subject. The selected dosage level will depend upon the activity of the therapeutic composition, the route of administration, combination with other drugs or treatments, the severity of the condition being treated, and the condition and prior medical history of the subject being treated. By way of example and not limitation, doses of compositions can be started at levels lower than required to achieve the desired therapeutic effect and to gradually increase the dosage until the desired effect is achieved. The potency of a composition can vary, and therefore an "effective amount" can vary.

After review of the disclosure of the presently disclosed subject matter presented herein, one of ordinary skill in the art can tailor the dosages to an individual subject, taking into account the particular formulation, method of administration to be used with the composition, and particular disease treated. Further calculations of dose can consider subject height and weight, gender, severity and stage of symptoms, and the presence of additional deleterious physical conditions.

By way of additional examples, the amount of active ingredient that may be combined with the carrier materials to produce a single dosage form will vary depending upon the subject to be treated and the particular mode of administration.

Citrulline is administered in some embodiments in a dose ranging from about 0.01 mg to about 1,000 mg, in some embodiments in a dose ranging from about 0.5 mg to about 500 mg, and in some embodiments in a dose ranging from about 1.0 mg to about 250 mg. Citrulline can also be administered in some embodiments in a dose ranging from about 100 mg to about 30,000 mg, and in some embodiments in a dose ranging from about 250 mg to about 1,000 mg. A representative dose is 3.8 g/m2/day of citrulline (molar equivalents, MW L-citrulline 175.2, MW L-arginine 174.2).

Representative intravenous citrulline solutions can comprise a 100 mg/ml (10%) solution. Representative intravenous citrulline dosages can comprise 200 mg/kg, 400 mg/kg, 600 mg/kg, and 800 mg/kg. In some embodiments, for example but not limited to a 600 or 800 mg/kg dosage, the dose can be decreased by an amount ranging from 50 mg/kg and 100 mg/kg to mitigate observed undesired effects on systemic blood pressure. In some embodiments, doses can be administered one or more times during a given period of time, such as a day.

In some embodiments a pharmaceutical composition comprises an amount of citrulline effective to raise plasma citrulline level to treat a condition as disclosed herein in a subject. In some embodiments, the level is determined by comparing plasma citrulline levels in a subject to be treated to that observed in a subject not suffering from the condition. In some embodiments, the amount of citrulline is effective to raise plasma citrulline level in a subject to at least 5 µmol/liter, optionally at least 10 µmol/liter, optionally at least 20 µmol/liter, optionally at least 25 µmol/liter, and optionally about 37 µmol/liter.

In some embodiments, the presently disclosed subject matter provides pharmaceutical compositions comprising citrulline and a pharmaceutically acceptable carrier, such as a pharmaceutically acceptable carrier in humans. In some embodiments, the presently disclosed subject matter provides pharmaceutical compositions comprising citrulline in dosages as described above.

A composition of the presently disclosed subject matter is typically administered orally or parenterally in dosage unit formulations containing standard nontoxic pharmaceutically acceptable carriers, adjuvants, and vehicles as desired. The term "parenteral" as used herein includes intravenous, intra-muscular, intra-arterial injection, or infusion techniques.

Injectable preparations, for example sterile injectable aqueous or oleaginous suspensions, are formulated according to the known art using suitable dispersing or wetting agents and suspending agents. The sterile injectable preparation can also be a sterile injectable solution or suspension in a nontoxic acceptable diluent or solvent, for example, as a solution in 1,3-butanediol.

Among the acceptable vehicles and solvents that can be employed are water, Ringer's solution, and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid find use in the preparation of injectables. Exemplary carriers include neutral saline solutions buffered with phosphate, lactate, Tris, and the like.

In a representative embodiment doses can be administered to a subject several times during a relevant treatment period, including but not limited to 1, 2, 3, 4, 5, 6 or more dosages.

It will be understood, however, that the specific dose level for any particular subject will depend upon a variety of factors including the age, body weight, general health, sex, diet, time of administration, route of administration, rate of excretion, drug combination and the severity of the particular disease undergoing therapy.

### EXAMPLES

The following Examples have been included to illustrate representavtive modes of the presently disclosed subject matter. In light of the present disclosure and the general level of skill in the art, those of skill will appreciate that the following Examples are intended to be exemplary only in that numerous changes, modification, and alterations can be employed without departing from the spirit and scope of the presently disclosed subject matter.

### EXAMPLES 1-4

The following Examples evaluate whether oral supplementation with L-citrulline during exposure of newborn piglets to 10 days of chronic hypoxia would prevent the development of pulmonary hypertension and the concomitant reduction in NO production.

### METHODS EMPLOYED IN EXAMPLES 1-4

### ANIMAL CARE

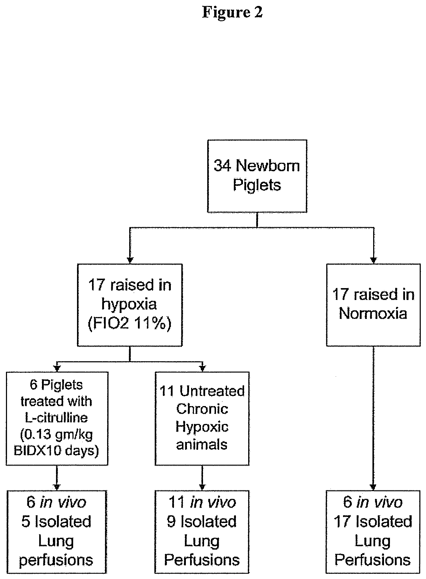

A total of 17 hypoxic and 17 control piglets were studied. See Figure 2. Control animals were studied on the day of arrival from the farm at 12 days of age. The hypoxic pigs (2 days old) were placed in a normobaric hypoxic chamber for 10 to 11 days. Normobaric hypoxia was provided using compressed air and nitrogen to create inspired oxygen of 8-11 % (PO₂ 60-72 Torr) and CO₂ was maintained at 3-6 Torr by absorption with soda lime. The animals were monitored with daily weights and physical exam twice daily. They were fed ad lib with sow milk replacer from a feeding device in the cage.

### L-CITRULLINE SUPPLEMENTATION

Six of the seventeen hypoxic piglets were supplemented with oral L-citrulline starting on the first day of the hypoxic exposure. *See* Figure 2. L-citrulline supplementation was provided at a dose of 0.13-gm/kilogram body weight twice a day using a syringe to deliver the dose orally. If it appeared to study personnel that the piglet had not ingested the majority of a dose, it was repeated. L-citrulline was mixed using a preparation (Sigma Pharmaceuticals, St. Louis, Missouri, United States of America, 98% purity) at a concentration of 0.13 grams per milliliter of distilled water and when completely dissolved passing this solution through a 0.20 Micron filter.

### IN VIVO HEMODYNAMICS

*In vivo* hemodynamics were measured in 6 of the control piglets and all of the hypoxic piglets. See Figure 2. For these measurements, the animals were weighed and then preanesthetized with Ketamine (15 mg/kg) and Acepromazine (2 mg/kg) intramuscularly. A tracheostomy, venous and arterial catheters, and thermistor were then placed as previously described using intravenous pentobarbital for sedation. Fike, C. D. et al., J Appl Physiol (2000) 88:1797-1803. Pulmonary artery pressure, left ventricular end diastolic pressure, and cardiac output were measured. Cardiac output was measured by a thermodilution technique (model 9520 thermodilution cardiac output computer, Edwards Laboratory, Irvine, California, United States of America) using a thermistor in the aortic arch and the left ventricle catheter as an injection port. Cardiac output was measured at end expiration as the mean of three injections of 3 ml of normal saline (0°C). Exhaled NO was measured as described below. During the *in vivo* measurements, animals were ventilated with room air using a piston-type ventilator at a tidal volume of 15-20 cc/kg, end-expiratory pressure of 2 mmHg, and a respiratory rate of 15-20 breaths per minute.

### EXHALED NITRIC OXIDE MEASUREMENT

For exhaled NO measurement in anesthetized animals, expiratory gas was sampled two to three times for 3 minute periods each and passed through a chemiluminescence analyzer (model 270B NOA; Sievers, Boulder, Colorado, United States of America) to measure NO concentration as previously described. Fike, C. D., et al., American Journal of Physiology (Lung, Cellular and Molecular Physiology 18) (1998) 274:L517-L526. Exhaled NO production (nmol/min) was calculated using minute ventilation and the measured exhaled NO concentration.

### ISOLATED LUNG PERFUSIONS

The lungs were isolated and perfused *in situ* with a Krebs Ringer bicarbonate (KRB) solution containing 5% dextran, mol. wt. 70,000, at 37°C and ventilated with a normoxic gas mixture (21% O₂ and 5% CO₂) as previously described. Fike, C. D. et al., J Appl Physiol (2000) 88:1797-1803. The lungs were perfused for 30-60 min until a stable pulmonary arterial pressure was achieved. Perfusate samples (1 ml) were then removed from the left atrial cannula every 10 min for a 60-minute period. The perfusate samples were centrifuged, and the supernatant was stored at -80°C for future analysis of nitrite/nitrate (NOx⁻) concentrations as described below. At the end of the perfusion, the volume of perfusate remaining in the circuit and reservoir was measured. In some cases, lung tissue was collected immediately following the perfusion, frozen with liquid nitrogen and then stored at -80 degrees for later measurement of eNOS content as described below.

### NOX⁻MEASUREMENT

A chemiluminescence analysis described previously was used to determine perfusate NOx⁻ concentration (nmol/ml) at each collection time. Fike, C. D. et al., J Appl Physiol (2000) 88:1797-1803; Turley, J. E. et al., Am J Physiol Lung Cell Mol Physiol (2003) 284:L489-L500 Perfusate (20 µl) was injected into the reaction chamber of a chemiluminescence NO analyzer (model 170B NOA, Sievers). The reaction chamber contained vanadium (III) chloride in 1 M HCl heated to 90°C to reduce nitrite and nitrate to NO gas. The NO gas was carried into the analyzer using a constant flow of N₂ gas via a gas bubble trap containing 1 M NaOH to remove HCl vapor. A standard curve was generated by adding known amounts of NaNO₃ to distilled water and assaying as described for the perfusion samples.

The perfusate NOx⁻ concentration (nmol/ml) was calculated for each collection time by multiplying the perfusate concentration of NOx⁻ at that sample collection time by the volume of the system (perfusion circuit + reservoir) at the sample collection time plus the amount of NOx⁻ removed with all previous samples. The rate of NOx⁻ production was determined from the slope of a linear regression line fit to the amount of NOx⁻ in the perfusate versus time for the first 60 minutes of the collection period.

### PLASMA AMINO ACID MEASUREMENTS

On the day of hemodynamic measurements and/or lung perfusion study, for control and both L-citrulline treated and untreated chronic hypoxic animals, blood was drawn prior to starting the study and the plasma frozen at -80 degrees for later determination of amino acid levels. For the L-citrulline treated hypoxic animals, the time of obtaining the blood sample was approximately 12 hours after the last dose of L-citrulline so was a trough level. In some of the L-citrulline treated animals (n=3), after blood sampling for a trough level, a dose of L-citrulline was given via nasogastric tube. Following this dose, blood samples were drawn every 30 minutes for 90 minutes (the length of the *in vivo* studies). All samples were spun, the plasma collected and frozen at -80 degrees for amino acid analysis.

Concentrations of plasma citrulline and arginine were determined by amino-acid analysis on protein-free extracts. Amino acids were separated by cation-exchange chromatography using a Hitachi L8800 amino acid analyzer (Hitachi USA, San Jose, California, United States of America). Calibration of the analyzer was performed before testing of piglet samples.

### WESTERN BLOT OF ENOS IN LUNG TISSUE

Using a standard immunoblot technique as previously described, we analyzed samples of whole lung homogenates from controls (n=3), untreated hypoxic (n=3) and L-citrulline treated hypoxic (n=3) animals for eNOS. We used 10 micrograms of total protein, a dilution of primary eNOS antibody of 1:500 (BD transduction) and a dilution of secondary anti-mouse antibody conjugated to horseradish peroxidase of 1:5000. Fike, C. D., et al., American Journal of Physiology (Lung, Cellular and Molecular Physiology 18) (1998) 274:L517-L526.

### CALCULATIONS AND STATISTICS

Pulmonary vascular resistance was calculated from the in vivo hemodynamic measurements: (Pulmonary arterial pressure-left ventricular end diastolic pressure) ÷ (Cardiac output/body weight).

Data are presented as means ± SE. The one-way ANOVA with Fisher's protected least significant difference (PLSD) post hoc comparison test was used to compare data between control, untreated hypoxic and L-citrulline treated hypoxic animals. A p-value less than 0.05 was considered significant. Meier, U., Pharm Stat (2006) 5:253-263.

### EXAMPLE 1

### IN VIVO HEMODYNAMIC MEASUREMENTS

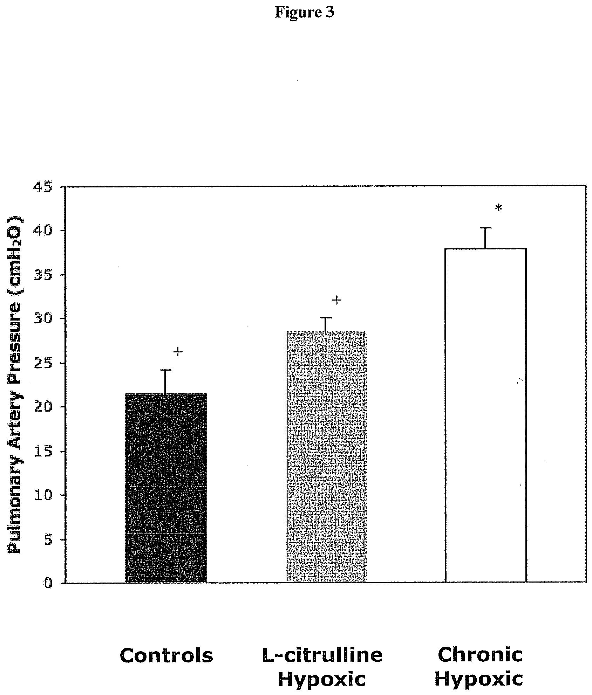

Both L-citrulline treated and untreated chronic hypoxic animals had lower cardiac output and weights and higher LVEDP measurements on the day of study at 12-13 days of age than comparable age control piglets (Table 1). Measurements of aortic pressure and blood gas indices were similar (paO₂ was 74±5 Torr in control piglets, 74±8 Torr in untreated hypoxic piglets and 78±7 Torr in L-citrulline treated hypoxic piglets; paCO2 was 39±2 in control piglets, 41±4 in untreated hypoxic piglets and 30±1.0 in L-citrulline treated hypoxic piglets) among groups. Notably, as shown in Figure 3, L-citrulline treated hypoxic animals had significantly lower pulmonary artery pressures than untreated hypoxic animals (p-value of 0.01). Pulmonary artery pressures did not differ between normoxic controls and L-citrulline treated hypoxic animals (p=0.08).

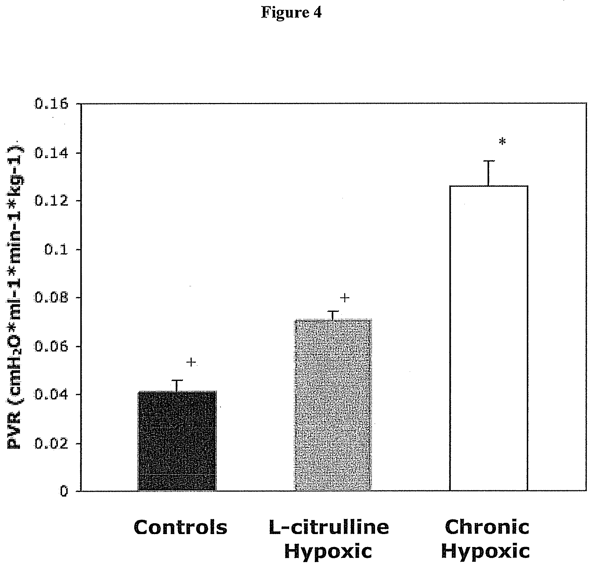

In addition, as shown in Figure 4, calculated pulmonary vascular resistance in those hypoxic animals treated with L-citrulline (0.071±0.003) were significantly lower than those of untreated hypoxic animals (p-value of 0.001). Furthermore, pulmonary vascular resistances were similar in L-citrulline treated hypoxic animals and normoxic controls (p-value of 0.07).

### EXAMPLE 2

### EXHALED NO OUTPUT AND PERFUSATE NOX⁻

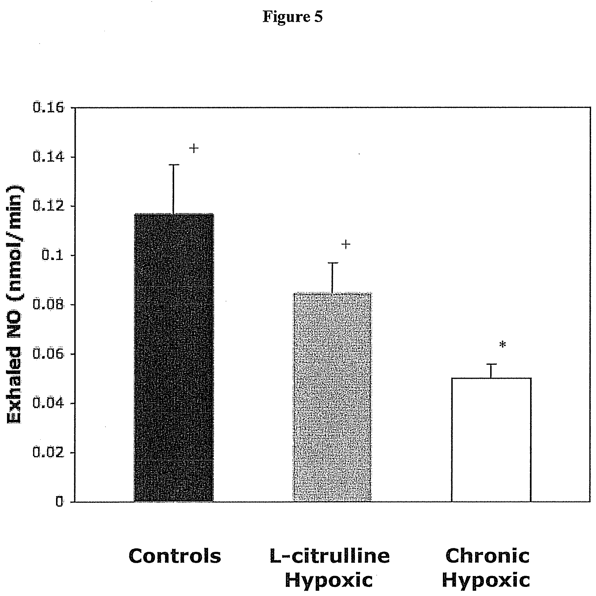

As shown in Figure 5, exhaled NO output in controls and L-citrulline treated hypoxic animals were higher than exhaled NO output in untreated hypoxic animals (p-values of 0.001 and 0.032 respectively). However, exhaled NO output did not differ between control and L-citrulline treated hypoxic animals (p=0.124).

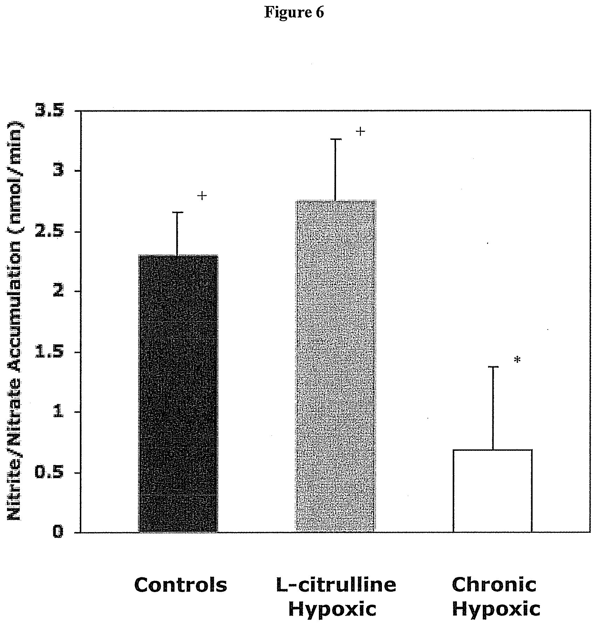

As shown in Figure 6, lungs from both the control (p=0.02) and L-citrulline treated hypoxic (p=0.04) animals had significantly higher NOx⁻ accumulation rates than lungs from untreated hypoxic animals. Furthermore, there was no difference in the rate of NOx⁻ accumulation between lungs from L-citrulline treated hypoxic animals and normoxic controls.

### EXAMPLE 3

### PLASMA AMINO ACIDS

As shown in Table 2, although not reaching statistical significance (p=0.05), plasma L-citrulline levels in untreated chronic hypoxic piglets were less than trough L-citrulline levels in treated hypoxic piglets. Moreover, when drawn ninety minutes after a dose, levels of L-citrulline in treated hypoxic animals were almost twice that of the untreated chronic hypoxic animals (p=0.001). However, regardless of the time the sample was drawn, plasma arginine levels were not higher in L-citrulline treated chronic hypoxic animals when compared to untreated hypoxic animals.

### EXAMPLE 4

### WESTERN BLOT FOR LUNG ENOS PROTEIN

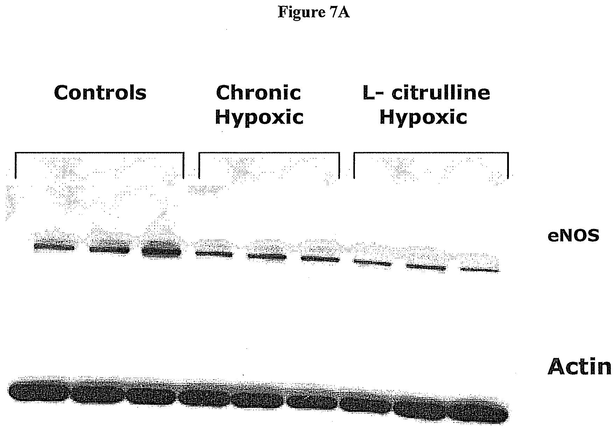

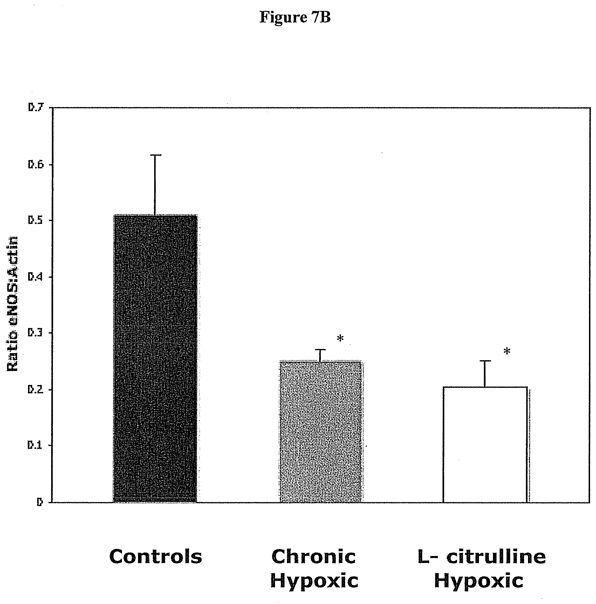

As shown in Figures 7A and 7B, the amount of eNOS protein present in the lung tissue of control animals was significantly higher than that present in the lungs of untreated hypoxic animals. Furthermore, the amount of eNOS protein present in the lung tissue of L-citrulline treated hypoxic piglets was not significantly different from that in the untreated hypoxic animals and was significantly lower than eNOS protein levels in control animals.

**TABLE 1**

| DATA FOR CONTROL, CHRONICALLY HYPOXIC AND L-CITRULLINE TREATED CHRONICALLY HYPOXIC PIGLETS | | | | | |
|---|---|---|---|---|---|
| N=number of animals, Values are means±SEM, *p<0.05 vs. controls, ANOVA with post-hoc comparison test. | | | | | |
| **Treatment Group** | **Weight at 12 days of age (kg)** | **Aortic Pressure (cm H₂O)** | **LVEDP (cmH₂0)** | **Cardiac Output (ml/min/kg)** | **Arterial pH** |
| Controls N=6 | 3.94±0.3 | 91±0.8 | 5.2±0.6 | 414±43 | 7.38±0.05 |
| Chronic Hypoxic N=11 | 2.76±0.15* | 100±4 | 7.4±0.5* | 244±16* | 7.38±0.01 |
| Citrulline Hypoxic N=6 | 2.6±0.09* | 97±6 | 7.2±0.4* | 270±41* | 7.36±0.02 |

**TABLE 2**

| PLASMA AMINO ACID LEVELS FOR CONTROL, CHRONICALLY HYPOXIC AND L-CITRULLINE TREATED CHRONICALLY HYPOXIC PIGLETS | | |
|---|---|---|
| Treatment Group | Citrulline | Arginine |
| Controls N=10 | 71±6 | 112±16 |
| Chronic Hypoxic N=8 | 111±23 | 51±10* |
| L-citrulline treated Hypoxic: trough N=6 | 161±5* | 39±10* |
| L-citrulline treated Hypoxic: 90 min. N=3 | 219±36*† | 43±5* |

N=number of animals, Values are means±SEM, *p<0.05 vs. controls, † p<0.05 vs. untreated chronic hypoxics, ANOVA with post-hoc comparison test, Citrulline Trough- plasma level approximately twelve hours after L-citrulline dose, Citrulline 90 min- plasma level 90 minutes after administration of L-citrulline dose.

### DISCUSSION OF EXAMPLES

In Examples 1-4, it was found that L-citrulline supplementation ameliorates the development of pulmonary hypertension in newborn piglets exposed to 10 days of chronic hypoxia. Other findings in this study are that both exhaled NO production and pulmonary vascular NOx⁻ accumulation rates are greater in L-citrulline-treated hypoxic piglets than in untreated hypoxic piglets. Thus, these findings show that L-citrulline supplementation significantly increased pulmonary NO production. The amount of eNOS protein was not increased in the treated hypoxic animals.

While it is not desired to be bound by any particular theory of operation, the mechanism by which L-citrulline mediates an increase in NO production is believed to be by increasing the amount of L-arginine available as a substrate for eNOS. Plasma levels of arginine in the L-citrulline treated animals in the present Examples were not significantly increased when compared with untreated hypoxic animals. This discordance between intracellular arginine and NO production, termed an "arginine paradox", appears to be present in view of the increase in NO production in the face of unchanged plasma arginine levels seen with L-citrulline supplementation in the present Examples. L-citrulline is a urea cycle intermediate metabolized to arginine by a recycling pathway of two enzymes, argininosuccinate synthase (AS) and argininosuccinate lyase (AL). These two enzymes, AS and AL, have been found co-located with eNOS in pulmonary endothelial cells. Boger, R. H., Curr Opin Clin Nutr and Met Care (2008) 11 :55-61. It is thought that together these enzymes produce a separate subcellular pool of arginine used exclusively for NO synthesis. Tissue and plasma arginine levels cannot accurately measure this subcellular pool.

L-citrulline could also have improved NO production and eNOS function by additional mechanisms. Again, while it is not desired to be bound by any particular theory of operation, another potential action of L-citrulline in the present Examples is the prevention of the uncoupling of eNOS by maintaining adequate levels of its substrate arginine.

Again, while it is not desired to be bound by any particular theory of operation, L-citrulline might also have affected the bioavailability of NO by compensating for increased NO degradation. During exposure to chronic hypoxia, superoxide production might increase from enzymatic sources other than eNOS, such as NADPH oxidase. Liu, et al., Am J Physiol Lung Cell Mol Physiol (2006) 290:L2-L10. This excess superoxide production might have directly interacted with NO to reduce its local production. In this case, it is possible that providing L-citrulline allowed enough NO production to compensate for the superoxide mediated reduction.

In summary, the present Examples show that L-citrulline ameliorates chronic hypoxia-induced pulmonary hypertension in newborn piglets. Also provided is evidence that the effectiveness of citrulline is due to increased NO production. Thus, L-citrulline is a useful therapy in neonates at risk of developing pulmonary hypertension due to chronic or intermittent unresolved hypoxia.

### REFERENCES

The references listed below as well as all references cited in the specification are incorporated herein by reference to the extent that they supplement, explain, provide a background for or teach methodology, techniques and/or compositions employed herein.
Jobe et al. (2001) Am J Respir Crit Care Med 163:1723-1729.
Ballard et al. (2006) N Engl J Med 355:343-353.
Ryan, R. M. (2006) J Perinatology 26:207-209.
Published U.S. patent application number US-2004-0235953-A1, published November 25, 2004.
PCT International Patent Application Publication No. WO 2005/082042, published September 9, 2005.
U.S. Patent No. 6,343,382.
U.S. Patent No. 6,743,823.

## Claims

1. A pharmaceutical composition comprising an amount of citrulline effective for use in treating subjects at risk for suffering or suffering from bronchopulmonary dysplasia.

2. The composition for use according to claim 1, wherein the composition is formulated for oral administration, intravenous administration, or a combination thereof.

3. The composition for use according to claim 1, wherein the subject is an infant.

4. The composition for use according to claim 3, wherein the infant is a preterm infant.

5. The composition for use according to any one of claims 1 to 4, wherein the citrulline is administered in a dose ranging from 100 mg to 30,000 mg.

6. The composition for use according to any one of claims 1 to 5, wherein the citrulline is administered in a dose ranging from 250 mg to 1,000 mg.

7. The composition for use according to any one of claims 1 to 6, wherein the subject suffers from hypocitrullinemia **characterized by** plasma citrulline levels of ≤ 37 µmol/liter.

8. The composition for use according to any one of claims 1-7, wherein the amount of citrulline is effective to raise plasma citrulline level in a subject to at least 5 µmol/liter, optionally at least 10 µmol/liter, optionally at least 20 µmol/liter, optionally at least 25 µmol/liter, and optionally 37 µmol/liter.

## Patentansprüche

1. Pharmazeutische Zusammensetzung, die eine Menge an Citrullin enthält, die für die Verwendung zur Behandlung von Subjekten wirksam ist, bei denen die Gefahr besteht, dass sie an bronchopulmonaler Dysplasie leiden werden, oder die daran bereits leiden.

2. Zusammensetzung zur Verwendung nach Anspruch 1, wobei die Zusammensetzung zur oralen Verabreichung, intravenösen Verabreichung oder einer Kombination davon formuliert ist.

3. Zusammensetzung zur Verwendung nach Anspruch 1, wobei das Subjekt ein Säugling ist.

4. Zusammensetzung zur Verwendung nach Anspruch 3, wobei der Säugling ein frühgeborener Säugling ist.

5. Zusammensetzung zur Verwendung nach einem der Ansprüche 1 bis 4, wobei das Citrullin in einer Dosis verabreicht wird, die im Bereich von 100 mg bis 30.000 mg liegt.

6. Zusammensetzung zur Verwendung nach einem der Ansprüche 1 bis 5, wobei das Citrullin in einer Dosis verabreicht wird, die im Bereich von 250 mg bis 1.000 mg liegt.

7. Zusammensetzung zur Verwendung nach einem der Ansprüche 1 bis 6, wobei das Subjekt an einer Hypocitrullinämie leidet, die durch Plasmacitrullinspiegel von ≤37 µmol/Liter gekennzeichnet sind.

8. Zusammensetzung zur Verwendung nach einem der Ansprüche 1 bis 6, wobei die Menge an Citrullin wirksam ist, um den Plasmacitrullinspiegel in einem Subjekt von mindestens 5 µmol/Liter, optional mindestens 10 µmol/Liter, optional mindestens 20 µmol/Liter, optional mindestens 25 µmol/Liter, und optional 37 µmol/Liter anzuheben.

## Revendications

1. Composition pharmaceutique comprenant une quantité de citrulline efficace destinée à être utilisée dans le traitement de sujets à risque de souffrir ou souffrant de dysplasie bronchopulmonaire.

2. Composition destinée à être utilisée selon la revendication 1, dans laquelle la composition est formulée pour une administration orale, une administration intraveineuse ou une de leurs combinaisons.

3. Composition destinée à être utilisée selon la revendication 1, dans laquelle le sujet est un enfant.

4. Composition destinée à être utilisée selon la revendication 3, dans laquelle l'enfant est un enfant de pré-terme.

5. Composition destinée à être utilisée selon l'une quelconque des revendications 1 à 4, dans laquelle la citrulline est administrée à une dose se situant dans la plage allant de 100 mg à 30000 mg.

6. Composition destinée à être utilisée selon l'une quelconque des revendications 1 à 5, dans laquelle la citrulline est administrée à une dose se situant dans la plage allant de 250 mg à 1000 mg.

7. Composition destinée à être utilisée selon l'une quelconque des revendications 1 à 6, dans laquelle le sujet souffre d'hypocitrullinémie **caractérisée par** des niveaux de citrulline plasmatique de ≤ 37 µmoles/litre.

8. Composition destinée à être utilisée selon l'une quelconque des revendications 1 à 7, dans laquelle la quantité de citrulline est efficace pour augmenter le niveau de citrulline plasmatique chez un sujet à au moins 5 mmoles/litre, éventuellement au moins 10 µmoles/litre, éventuellement au moins 20 µmoles/litre, éventuellement au moins 25 µmoles/litre et éventuellement au moins 37 µmoles/litre.
